(19) **Europäisches Patentamt**

**European Patent Office**

**Office européen des brevets**

(11) **EP 0 556 112 B1**

(12) **FASCICULE DE BREVET EUROPEEN**

(45) Date de publication et mention
de la délivrance du brevet:
**30.07.1997 Bulletin 1997/31**

(51) Int. Cl.⁶: **A61N 1/30**

(21) Numéro de dépôt: **93400331.0**

(22) Date de dépôt: **10.02.1993**

(54) **Dispositif d'ionophorèse pour l'administration transcutanée d'une quantité totale donnée d'un principe actif à un sujet**

Iontophoreseeinrichtung zur transdermalen Verabreichung einer vorgegebenen Gesamtmenge von aktiven Grundstoffen an ein Lebewesen

Iontophoresis device for transdermal delivery of a given total amount of an active agent to a subject

(84) Etats contractants désignés:
**CH DE ES GB IT LI NL SE**

(30) Priorité: **14.02.1992 FR 9201678**

(43) Date de publication de la demande:
**18.08.1993 Bulletin 1993/33**

(73) Titulaires:
• **ELF AQUITAINE**
**92400 Courbevoie (FR)**
• **ELF SANOFI**
**75008 Paris (FR)**

(72) Inventeurs:
• **Muller, Daniel**
**F-64000 Pau (FR)**

• **Simonin, Jean-Pierre**
**F-75009 Paris (FR)**

(74) Mandataire: **Boillot, Marc**
**Elf Aquitaine Production,**
**Département Propriété Industrielle,**
**Tour Elf,**
**Cédex 45**
**92078 Paris La Défense (FR)**

(56) Documents cités:
EP-A- 0 182 520          WO-A-90/04432
WO-A-91/16946          FR-A- 2 509 182
US-A- 4 167 190          US-A- 4 764 164

## Description

L'invention concerne un dispositif d'ionophorèse pour l'administration transcutanée d'une quantité totale donnée d'un principe actif à un sujet.

Dans le traitement courant de nombreuses affections, il est nécessaire d'administrer un médicament ou autre principe actif à un sujet de manière contrôlée et souvent prolongée. Parmi les nombreuses techniques mises à la disposition du galéniste, celle de l'ionophorèse représente une alternative intéressante pour le contrôle de l'administration de principes actifs tels que des substances médicamenteuses dans l'organisme du sujet. Une telle technique consiste à utiliser le courant électrique pour contrôler la quantité mais aussi la vitesse de délivrance d'un principe actif à travers la peau d'un sujet. Dans de nombreux cas, cette technique s'avère très efficace en augmentant de manière significative l'apport de principe actif dû au courant, comparativement à la quantité délivrée sans courant.

L'administration transcutanée d'un principe actif par ionophorèse à un sujet est généralement réalisée, à partir d'une solution aqueuse ou d'un gel aqueux renfermant le principe actif sous une forme au moins partiellement ionisée ou sous une forme neutre, en appliquant un signal électrique entre, d'une part, une première électrode, dite électrode active, ayant même polarité que les ions du principe actif à administrer ou une polarité positive si le principe actif est neutre et se trouvant en contact avec un élément réservoir, qui renferme le principe actif et se trouve placé au contact d'une première zone de la peau du sujet, et, d'autre part, une deuxième électrode, dite contre-électrode ou électrode passive, de polarité opposée à celle associée au principe actif, qui est placée, directement ou par le biais d'un électrolyte indifférent, au contact d'une deuxième zone de la peau du sujet distincte de la première zone. Lors du passage du courant, généré par application de la tension entre les électrodes, dans le circuit ainsi réalisé, les ions du principe actif migrent, à l'opposé de l'électrode de même polarité (électrode active), à travers la peau et les tissus du sujet vers l'électrode de polarité opposée (contre-électrode) et se retrouvent ainsi à passer dans le système circulatoire du sujet. De même, les molécules neutres de principe actif sont entraînées, à l'opposé de l'électrode positive, dans le flux aqueux d'électroosmose à travers la peau et les tissus du sujet vers l'électrode négative (contre-électrode) et se retrouvent également ainsi à passer dans le système circulatoire du sujet.

Une technique connue pour administrer une quantité totale donnée de principe actif par ionophorèse à un sujet consiste à opérer en imposant un courant d'intensité I constante et à arrêter le traitement au bout d'une durée t telle que la quantité d'électricité $Q = I \times t$ corresponde à la quantité théorique d'électricité nécessaire pour administrer la quantité totale donnée de principe actif. Dans une telle technique, le maintien de l'intensité du courant à une valeur constante peut entraîner une variation substantielle de la tension électrique appliquée entre les électrodes, du fait que l'impédance de la peau n'est pas constante dans le temps et de plus peut varier fortement d'un individu à l'autre et, pour un même individu, d'une zone d'administration du principe actif à l'autre. Il s'ensuit l'apparition de phénomènes d'intolérance, de brûlures, voire de destruction par oxydation ou réduction du ou des principes actifs à administrer avec formation de composés secondaires, ainsi que des variations importantes de pH souvent mal tolérées. Il est alors nécessaire d'ajouter des dispositifs de sécurité limiteurs de tension aux circuits électroniques utilisés pour générer le courant d'intensité constante dans le circuit d'ionophorèse, lesdits dispositifs, outre le surcoût qu'ils entraînent, constituant un risque supplémentaire de panne ou d'accident.

Une autre technique connue pour administrer une quantité totale donnée de principe actif par ionophorèse à un sujet consiste à opérer en imposant une tension électrique constante entre les électrodes et en mesurant en continu la quantité d'électricité utilisée depuis le début du traitement, et à arrêter ledit traitement lorsque ladite quantité d'électricité atteint une valeur correspondant à celle de la quantité théorique d'électricité nécessaire pour administrer la quantité totale donnée de principe actif. L'inconvénient majeur de cette technique réside dans la nécessité de faire appel à un coulomètre pour mesurer la quantité d'électricité utilisée lors du traitement, car la présence d'un tel appareil complique la structure des circuits électroniques du dispositif d'ionophorèse et rend plus onéreuses la réalisation de ce dispositif et sa mise en oeuvre. De plus, l'utilisation d'un coulomètre est inadaptée à l'emploi de courants pulsés.

Les Demanderesses ont observé que lorsque, d'une part, le milieu renfermant la solution de principe actif connecté à l'électrode active et le milieu renfermant l'électrolyte indifférent relié à la contre-électrode et, d'autre part, les signaux électriques appliqués aux systèmes ionophorétiques sont bien définis, les quantités totales de principes actifs diffusant à travers la peau d'un sujet sont peu dépendantes des densités de courant, très variables d'un sujet à l'autre ou d'un traitement à l'autre, mais sont en relation directe avec la quantité totale de courant ayant circulé entre les électrodes. En d'autres termes, le rendement électrique, c'est-à-dire la quantité de principe actif ayant diffusé à travers la peau sous l'action d'une même quantité de courant, dépend des milieux réactionnels et des signaux appliqués entre les électrodes mais très peu des sujets. En s'appuyant sur ces observations, les Demanderesses ont trouvé que l'on pouvait très simplement administrer une quantité totale donnée de principe actif à travers la peau d'un sujet par ionophorèse en constituant au moins l'une des électrodes du système ionophorétique par une électrode consommable conduisant, après passage d'une quantité prédéterminée de courant, soit à une surtension importante par rapport à celle de son état initial ou encore à une rupture de la chaîne électrochimique mise en jeu de telle sorte que ledit courant soit pratiquement interrompu.

En opérant selon l'invention, on peut effectuer l'administration transcutanée de quantités totales données de principe actif par ionophorèse à un sujet sans rencontrer les inconvénients des techniques antérieures connues rappelées brièvement plus haut.

L'invention a donc pour objet un dispositif d'ionophorèse pour l'administration transcutanée d'une quantité totale donnée d'un principe actif à un sujet, ledit dispositif étant du type de celui décrit dans la citation WO-A-9116946 et comportant donc un premier ensemble électrode constitué d'une première électrode, dite électrode active, en contact avec un élément réservoir qui, d'une part, contient un électrolyte renfermant le principe actif sous une forme au moins partiellement ionisée ou sous une forme neutre et en quantité prédéterminée supérieure à la quantité totale donnée à administrer au sujet et, qui, d'autre part, est adapté pour assurer, lorsqu'il est placé au contact d'une zone de la peau du sujet un continuum conducteur ionique entre ladite première électrode et ladite zone, ladite première électrode ayant même polarité que les ions du principe actif ou bien est positive si le principe actif est neutre, un deuxième ensemble électrode constitué soit (i) d'une deuxième électrode, dite contre-électrode, de polarité opposée à celle associée au principe actif, ou bien, de préférence, (ii) d'une telle deuxième électrode en contact avec un élément récepteur renfermant un électrolyte indifférent, ledit élément récepteur étant agencé pour assurer, lorsqu'il est placé au contact d'une portion de la peau du sujet, un continuum conducteur ionique entre la deuxième électrode et ladite portion, et un générateur de signaux électriques relié aux deux électrodes, la première électrode en contact avec l'élément réservoir et/ou la deuxième électrode en contact avec l'élément récepteur étant une électrode consommable formée d'une matière électrochimiquement consommable associée soit à un support isolant, soit à un support conducteur électronique, ledit support conducteur électronique étant réalisé en un matériau qui résiste à la corrosion par l'électrolyte associé à l'électrode en l'absence de courant et qui présente, lorsque l'électrode consommable est du type cathode, une surtension d'hydrogène en présence dudit électrolyte au moins égale à celle de l'aluminium ou bien qui n'est pas consommable par oxydation électrochimique lorsque l'électrode consommable est du type anode. L'électrode consommable ou l'une des électrodes consommables renferme une quantité limitée de matière consommable électrochimiquement associée au support. Selon l'invention le dispositif du type précité se caractérise en ce que ladite quantité limitée étant choisie pour que la quantité d'électricité nécessaire à sa consommation électrochimique soit inférieure à la quantité d'électricité nécessaire pour administrer la quantité prédéterminée de principe actif renfermée dans l'électrolyte, de telle sorte que la circulation du courant entre les électrodes soit pratiquement interrompue lorsque la matière consommable de l'électrode portant la quantité limitée de matière consommable et dite électrode consommable limitante, a été consommée et de telle sorte que la quantité d'électricité nécessaire à la consommation de la quantité limitée de matière consommable puisse correspondre à la quantité d'électricité nécessaire pour administrer la quantité totale donnée de principe actif au sujet.

Si l'élément réservoir ne renfermait, au début de l'opération, qu'une quantité de principe actif égale à la quantité totale donnée de principe actif à administrer et si l'on faisait passer le courant jusqu'à ce que cette quantité ait diffusé entièrement à travers la peau du sujet, ledit courant, vers la fin de l'opération, servirait surtout à transporter les ions autres que ceux du principe actif, ce qui entraînerait une consommation excessive d'énergie et des durées de traitement importantes. Il est donc préférable, pour éviter les inconvénients ci-dessus, que la quantité de principe actif présente dans l'élément réservoir au début de l'opération soit en excès par rapport à ladite quantité totale donnée, ledit excès pouvant être, par exemple, d'environ 2 % à 1000 % et plus spécialement d'environ 2% à 500% de cette quantité totale donnée.

L'électrode, qui est formée par l'association de la quantité limitée de la matière consommable électrochimiquement et du support, à savoir support conducteur électronique ayant les caractéristiques précitées ou bien support isolant, et que l'on désigne ici par "électrode consommable limitante", peut être utilisée comme électrode active ou comme contre-électrode, et peut selon le cas être du type anode ou bien du type cathode. Dans l'un ou l'autre cas, on peut utiliser une électrode active du type électrode consommable limitante associée à une contre-électrode non consommable électrochimiquement ou bien encore employer une électrode active non consommable électrochimiquement associée à une contre-électrode du type électrode consommable limitante. On peut également utiliser une électrode active et une contre-électrode, qui toutes les deux sont des électrodes consommables, l'une desdites électrodes étant une électrode consommable limitante, tandis que l'autre électrode est une électrode consommable non limitante. Par électrode consommable non limitante, on entend une électrode renfermant une matière consommable électrochimiquement en quantité en excès par rapport à la quantité qui serait consommée par le passage de la quantité d'électricité consommant la quantité de matière consommable électrochimiquement de l'électrode consommable limitante.

Dans une électrode consommable de type cathode, qu'elle soit une électrode consommable limitante ou une électrode consommable non limitante, la matière consommable électrochimiquement se consomme par réduction. Elle peut être choisie avantageusement parmi les composés métalliques ionisables dont les ions métalliques sont susceptibles d'être réduits électrochimiquement en le métal correspondant. Parmi ces composés métalliques on peut citer, à titre non limitatif, les composés AgCl et CuCl.

Dans une électrode consommable de type anode, qu'elle soit une électrode consommable limitante ou une électrode consommable non limitante, la matière consommable électrochimiquement se consomme par oxydation. Elle peut être choisie parmi les métaux se consommant par oxydation électrochimique, et notamment parmi les métaux tels que Al, Cu, Mg, Zn et Ag.

Le support de l'électrode consommable limitante peut être en un matériau isolant et notamment en un matériau plastique organique isolant tel que polypropylène, polyéthylène, PVC, polyamide ou bien en un matériau conducteur électronique métallique ou non métallique ayant les caractéristiques définies précédemment, ledit matériau conducteur électronique pouvant être avantageusement tel que titane, aluminium, argent, tantale, vanadium, acier inoxydable, zinc, carbone, graphite ou polymère conducteur, lorsque l'électrode consommable limitante est la cathode, ou bien tel que platine, titane, acier inoxydable, or, carbone, graphite et polymère conducteur lorsque l'électrode limitante est l'anode. L'électrode consommable non limitante peut avoir également une structure comparable à celle de l'électrode consommable limitante et peut donc comporter un support tel que défini ci-dessus.

A titre d'exemples non limitatifs d'électrodes consommables limitantes ou d'électrodes consommables non limitantes, utilisables comme cathodes dans le dispositif selon l'invention, on peut citer les électrodes à base d'AgCl ou de CuCl sur un support d'argent, de cuivre, d'acier inoxydable, de titane, de carbone, de polypropylène, de polyéthylène ou d'un polymère conducteur. Comme exemples d'électrodes consommables limitantes ou d'électrodes consommables non limitantes, utilisables comme anodes dans le dispositif selon l'invention, on peut mentionner, à titre non limitatif, les électrodes non limitantes à base d'un métal consommable par oxydation électrochimique choisi parmi Al, Ag, Cu, Mg et Zn et les électrodes limitantes à base d'un tel métal déposé sur un support isolant tel que polypropylène ou polyéthylène ou sur un support conducteur électronique choisi parmi titane, acier inoxydable, platine, carbone, graphite et polymère conducteur.

Comme indiqué précédemment, la matière consommable électrochimiquement de l'électrode consommable limitante est présente dans ladite électrode en quantité telle que la quantité d'électricité nécessaire à sa consommation électrochimique corresponde à la quantité d'électricité à utiliser pour administrer la quantité totale donnée du principe actif au sujet. Cette dernière quantité d'électricité, qui dépend essentiellement du système ionophorétique employé, c'est-à-dire des milieux réactionnels en contact avec l'électrode active et la contre-électrode, du signal électrique appliqué aux électrodes et de la nature desdites électrodes, est déterminée par le biais d'essais préalables pour chaque type de système ionophorétique mis en oeuvre.

Les électrodes non consommables électrochimiquement que l'on peut utiliser comme électrodes actives ou comme contre-électrodes dans le dispositif selon l'invention peuvent être choisies parmi les électrodes non consommables électrochimiquement utilisées habituellement en ionophorèse. En particulier, on peut faire appel à des électrodes en carbone, platine, titane, acier inoxydable, graphite, polymère conducteur.

Le générateur électrique applique entre l'électrode active et la contre électrode un signal électrique qui peut être soit un signal intensiométrique, c'est-à-dire un signal d'intensité moyenne imposée, par exemple constante (signal intensiostatique), soit, de préférence, un signal potentiométrique, c'est-à-dire un signal de tension moyenne imposée, par exemple constante (signal potentiostatique). Le signal électrique de type intensiostatique ou de type potentiostatique peut être continu ou pulsé et permanent ou intermittent, avec ou sans inversion temporaire de polarité. Sa fréquence peut aller de 0 à 500 kHz et plus particulièrement de 0 à 100 kHz. Lorsque le signal électrique est d'un type pulsé, il peut avoir un rapport cyclique, c'est-à-dire un rapport entre la durée de l'impulsion élémentaire, dont la répétition forme le signal pulsé, et l'intervalle de temps séparant deux apparitions successives de cette impulsion, allant de 0,05 à 0,95 et plus particulièrement de 0,1 à 0,8.

Avantageusement, la tension moyenne du signal appliqué entre l'électrode active et la contre-électrode est choisie entre 0,1 et 50 Volts et plus spécialement entre 0,5 et 20 volts de telle sorte que la densité du courant moyen généré entre lesdites électrodes ait une valeur inférieure à 5mA/cm$^2$ et plus particulièrement inférieure ou égale à 1mA/cm$^2$ et, par exemple comprise entre 0,03 et 0,5mA/cm$^2$.

Le générateur de signaux électriques du dispositif selon l'invention peut être de tout type connu permettant de générer des signaux électriques d'intensité moyenne imposée ou de tension moyenne imposée, qui sont continus ou pulsés et permanents ou intermittents, avec ou sans inversion temporaire de polarité, et qui présentent les caractéristiques définies ci-dessus.

L'électrolyte, qui est présent dans l'élément réservoir en contact avec l'électrode active, contient avantageusement une solution aqueuse ou un gel aqueux, adhésif ou non, qui renferme le principe actif à administrer sous une forme au moins partiellement ionisée ou sous une forme neutre. De même, l'électrolyte indifférent, qui est éventuellement en contact avec la contre-électrode, se présente, au moins en partie, sous la forme d'une solution aqueuse ou d'un gel aqueux adhésif ou non. Ces solutions ou gels aqueux peuvent constituer la totalité de l'électrolyte présent dans l'élément réservoir ou de l'électrolyte indifférent ou bien peuvent former une partie seulement desdits électrolytes et être alors dispersés dans un milieu non aqueux formant le reste de l'électrolyte et choisi pour ne pas interrompre le continuum conducteur ionique entre l'électrode et la peau et pour accroître la qualité de l'adhésion entre l'électrode et la peau. Ces solutions aqueuses ou gels aqueux peuvent être obtenus comme il est bien connu dans les techniques d'ionophorèse. Des exemples de gels aqueux ou de solutions aqueuses épaissies sont notamment décrits respectivement dans les citations US-A-4 764 164 et US-A-3 163 166.

Le milieu aqueux renfermant le principe actif, de même que le milieu aqueux constituant l'électrolyte indifférent peuvent renfermer, comme il est bien connu en ionophorèse, des agents tampons susceptibles de contrôler le pH desdits milieux. On peut encore contrôler le pH desdits milieux aqueux sans faire appel à des agents tampons, en utilisant une électrode consommable réversible, ce qui permet d'éviter ou tout au moins de réduire fortement l'introduction d'ions étrangers dans la peau à partir du milieu aqueux renfermant le principe actif.

Le dispositif d'ionophorèse selon l'invention permet d'administrer par voie transcutanée à un sujet des principes actifs divers et, notamment, des molécules thérapeutiques telles que, par exemple, insuline, metoprolol, hydrocodone, tetracyclines, salbutamol, acide valproique, propranolol, arginine-desmopressine, despo- pressine ou autres.

Le dispositif selon l'invention peut être réalisé à partir de tout dispositif d'ionophorèse connu, que l'on a modifié pour remplacer l'une de ses électrodes, à savoir électrode active ou contre-électrode, par une électrode consommable limitante selon l'invention, c'est-à-dire telle que définie dans ce qui précède.

En particulier, le dispositif selon l'invention peut être un dispositif autonome portable, à fixer par bracelet ou éventuellement à coller sur la peau, comportant des électrodes ayant chacune une aire inférieure à $50cm^2$ et plus particulièrement comprise entre 1 et $40cm^2$ et un générateur de signaux électriques miniaturisé. Ainsi, un dispositif portable autonome selon l'invention peut avoir une structure analogue à celle des dispositifs d'ionophorèse portables autonomes décrits, par exemple, dans les citations US-A-4325367, US-A-4557723, EP-A-0060452 et FR-A-2509182 sous réserve que l'une des électrodes dudit dispositif soit une électrode consommable limitante selon l'invention, lesdites électrodes limitante et non limitante ayant chacune une aire inférieure à $50cm^2$ et plus particulièrement comprise entre 1 et $40cm^2$. Par exemple, on peut faire appel à une électrode consommable limitante du type cathode à base d'AgCl ou de CuCl sur un support d'argent, de cuivre, de carbone, de polypropylène, de polyéthylène ou d'un polymère conducteur, l'électrode non limitante, à savoir l'anode, pouvant être une anode conventionnelle, par exemple anode en un métal ou alliage métallique tel que titane, platine, acier inoxydable ou encore en un matériau conducteur électronique non métallique tel que carbone ou graphite, ou bien encore une anode consommable non réversible ou réversible, par exemple anode en un métal tel que Al, Cu, Mg, Zn et Ag éventuellement déposée sur un support isolant tel que polypropylène ou polyéthylène ou bien sur un support conducteur électronique choisi parmi titane, acier inoxydable, platine, carbone, graphite et polymère conducteur. On peut également faire appel à une électrode consommable limitante du type anode, par exemple, à base d'un métal choisi parmi Al, Ag, Cu, Mg et Zn déposé sur un support de polypropylène, polyéthylène, titane, acier inoxydable, platine, carbone, graphite ou polymère conducteur, l'électrode non limitante, à savoir la cathode, pouvant être une cathode conventionnelle, par exemple cathode en carbone, graphite, titane, acier inoxydable ou polymère conducteur ou bien encore une cathode consommable non limitante, par exemple à base d'AgCl ou de CuCl sur un support d'argent, de cuivre, de carbone, de polypropylène, de polyéthylène ou d'un polymère conducteur.

L'invention est illustrée par les exemples suivants donnés à titre non limitatif.


## EXEMPLE 1

### Etude du passage transdermique de fluorescéine sous forme de sel de sodium par ionophorèse.

On opérait dans des cellules d'ionophorèse de structure identique. Chaque cellule d'ionophorèse était constituée de deux compartiments cylindriques adjacents coaxiaux de $2cm^2$ de section transversale à savoir, un compartiment donneur et un compartiment receveur ou compartiment de contre-électrode, ces deux compartiments étant séparés l'un de l'autre, de façon étanche, par un morceau de peau de rat nu (OFA hr/hr) servant de membrane pour l'étude de la diffusion transcutanée. Le compartiment donneur, d'un volume de 3ml, renfermait une solution aqueuse à 0,1% en poids de sel de sodium de la fluorescéine et était équipé d'un barreau magnétique permettant l'homogénéisation de la solution. Le compartiment receveur ou de contre-électrode, identique au compartiment donneur, renfermait du sérum physiologique additionné de 500 ppm en poids de $NaN_3$ et était également agité à l'aide d'un barreau magnétique. A son extrémité opposée au compartiment receveur, le compartiment donneur était équipé d'une électrode constituée d'un film d'argent pur de $25\mu m$ d'épaisseur et de $2cm^2$ de surface active préalablement chloruré. Cette chloruration était réalisée électrochimiquement par passage d'un courant continu de 10mA alors que ledit film d'argent était plongé dans un bain d'acide chlorhydrique 0,1N et constituait le pôle positif par rapport à une électrode de cuivre également plongée dans le même bain de HCl, la quantité de courant étant contrôlée, au moyen d'un coulomètre monté dans le circuit, pour former la quantité désirée d'AgCl sur le film d'argent. Le compartiment de contre-électrode était équipé d'une électrode de $2cm^2$ en argent légèrement chloruré (quantité d'AgCl correspondant à 0,1 coulomb/$cm^2$). La face chlorurée de chaque électrode était tournée du côté de la membrane en peau de rat.

Les échantillons de peau de rat avaient été débarrassés des tissus sous-cutanés et conservés par congélation à -40°C jusqu'à leur montage dans la cellule d'ionophorèse, faces dermiques tournées vers le compartiment receveur, après un passage de 15 minutes à température ambiante dans du sérum physiologique additionné de 0,05% en poids de $NaN_3$.

Pour chacun des essais réalisés, quatre cellules d'ionophorèse identiques (cellules A, B, C et D) étaient mise en route simultanément. La surface effective d'échange était de 2 $cm^2$ pour chaque peau.

Un générateur de courant pul sé permetta it d'établir simultanément entre les électrodes des quatre cellules montées en parallèle un signal électrique de type potentiométrique de tension crête égale à 4 volts avec un rapport cyclique de 30 % et une fréquence de 20 kHz. Une résistance de 100 ohms était branchée en série dans le circuit.

Le suivi de la tension aux bornes de cette résistance permettait de déterminer l'instant à partir duquel le chlorure d'argent était consommé en totalité, ledit instant étant indiqué par une chute brutale de ladite tension.

Le courant pulsé généré par le générateur était appliqué pendant 6 heures, le compartiment donneur de chaque cellule contenant la fluorescéine étant relié au pôle négatif dudit générateur et les contre-électrodes des cellules au pôle positif.

Au bout de ladite durée, on prélevait le milieu contenu dans le compartiment receveur et déterminait, par dosage par fluorescence, la quantité de fluorescéine ayant traversé la peau séparant les compartiments donneur et receveur de chaque cellule.

Cinq essais 1a à 1e ont été réalisés comme suit :

- Essai 1a : Aucun courant n'a été appliqué aux électrodes dans le but de déterminer la diffusion transcutanée passive sur 6 heures.
- Essai 1b : L'électrode d'argent chloruré présente dans le compartiment donneur renfermait une quantité d'AgCl correspondant à 20 coulombs, c'est-à-dire très supérieure à la quantité susceptible d'être consommée pendant la durée de mise en oeuvre (6 heures) de l'opération d'ionophorèse (électrode non limitante)
- Essais 1c à 1e : L'électrode d'argent chloruré présente dans le compartiment donneur renfermait une quantité d'AgCl correspondant respectivement à 1 coulomb (essai 1c), 2 coulombs (essai 1d) et 4 coulombs (essai 1e), c'est-à-dire inférieure à la quantité susceptible d'être consommée pendant la durée de mise en oeuvre (6 heures) de l'opération d'ionophorèse (électrode limitante).

Pour chacun des essais, on a déterminé pour chaque cellule :

- la quantité totale Q de fluorescéine ayant diffusé en 6 heures dans le compartiment receveur ;
- la durée d'activité de l'électrode du compartiment donneur, c'est-à-dire la durée de l'opération d'ionophorèse dans le cas des essais 1a et 1b sans électrode limitante ou bien la durée au bout de laquelle le chlorure d'argent de l'électrode limitante a été consommé dans le cas des essais 1c et 1e avec électrode limitante ;
- le flux F représentant le quotient de la quantité Q par la durée d'activité et par la surface de l'électrode ; et
- le rapport Q/n de la quantité Q à la quantité de chlorure d'argent consommée, cette dernière quantité étant exprimée en coulombs.

Les résultats obtenus sont rassemblés dans le tableau I.

TABLEAU I

| Essai 1a | Diffusion transcutanée passive sur 6 heures | | | | | | |
|---|---|---|---|---|---|---|---|
| | Cell.A | Cell.B | Cell.C | Cell.D | Moyenne | Ecart type | %de variation |
| Q ($\mu$g) | 0,5 | 1,2 | 0,2 | 1,4 | 0,82 | 0,49 | 50 % |
| F($\mu$g/cm$^2$.h) | 0,04 | 0,1 | 0,02 | 0,12 | 0,07 | 0,04 | 50 % |
| Essai 1b | Ionophorèse sur 6 heures -Electrodes réversibles non limitantes | | | | | | |
| | Cell.A | Cell.B | Cell.C | Cell.D | Moyenne | Ecart type | %de variation |
| Q ($\mu$g) | 76,8 | 62 | 198,4 | 135,6 | 118,3 | 53,4 | 46 % |
| F($\mu$g/cm$^2$.h) | 6,4 | 5,17 | 16,5 | 11,3 | 9,85 | 4,49 | 46 % |
| Essai 1c | Ionophorèse sur 6 heures -Electrodes Ag/AgCl à 1 C d'AgCl | | | | | | |
| | Cell.A | Cell.B | Cell.C | Cell.D | Moyenne | Ecart type | %de variation |
| Q ($\mu$g) | 15,55 | 20,1 | 16,75 | 19,7 | 18,02 | 2,1 | 10,7 % |
| Durée d'activité (h) | 4,5 | 1,16 | 0,83 | 2,5 | 2,25 | 1,43 | 64 % |
| F ($\mu$g/cm$^2$.h) | 1,73 | 8,66 | 10,1 | 3,94 | 6,11 | 3,4 | 56 % |
| Q/n ($\mu$g/C) | 15,55 | 20,1 | 16,75 | 19,7 | 18,02 | 2,1 | 10,7 % |
| Essai 1d | Ionophorèse sur 6 heures -Electrodes Ag/AgCl à 2 C d'AgCl | | | | | | |
| | Cell.A | Cell.B | Cell.C | Cell.D | Moyenne | Ecart type | %de variation |
| Q ($\mu$g) | 33,1 | 39,4 | 35 | 37,4 | 36,22 | 2,38 | 6,6 % |
| Durée d'activité (h) | 4,83 | 2,5 | 2,75 | 2,25 | 3,08 | 1,03 | 33 % |
| F ($\mu$g/cm$^2$.h) | 3,42 | 7,88 | 6,36 | 8,31 | 6,49 | 1,92 | 29,5 % |
| Q/n ($\mu$g/C) | 16,55 | 19,7 | 17,5 | 18,7 | 18,11 | 1,19 | 6,6 % |
| Essai 1e | Ionophorèse sur 6 heures -Electrodes Ag/AgCl à 4 C d'AgCl | | | | | | |
| | Cell.A | Cell.B | Cell.C | Cell.D | Moyenne | Ecart type | %de variation |
| Q ($\mu$g) | 80,7 | 67,9 | 74,5 | 66,3 | 72,35 | 7,01 | 7,9 % |
| Durée d'activité (h) | 4,41 | 5,5 | 2,66 | 6 | 4,65 | 1,28 | 7,9 % |
| F ($\mu$g/cm$^2$.h) | 9,15 | 6,17 | 12,05 | 5,53 | 8,21 | 2,59 | 31,4 % |
| Q/n ($\mu$g/C) | 20,18 | 17 | 18,6 | 16,6 | 18,1 | 1,43 | 7,9 % |

EXEMPLE 2 :

Etude du passage transdermique du valproate de sodium (isooctanoate de sodium)

On opérait dans des cellules d'ionophorèse ayant chacune une structure identique à celle des cellules utilisées dans l'exemple 1. Dans chaque cellule, le compartiment donneur était rempli d'une solution aqueuse de valproate de sodium à 5 % en poids, ladite solution étant additionnée de 500 ppm de NaN$_3$ à titre d'agent bactéricide, tandis que le compartiment receveur ou compartiment de contre-électrode était rempli du même liquide que celui utilisé dans l'exemple 1. A son extrémité opposée au compartiment receveur, le compartiment donneur était équipé d'une électrode constituée d'un film d'argent pur de 25 $\mu$m d'épaisseur et de 2 cm$^2$ de surface active préalablement chloruré, la chloruration étant réalisée comme indiqué dans l'exemple 1, tandis que le compartiment de contre-électrode était équipé d'une électrode de 2 cm$^2$ en argent légèrement chloruré (quantité de chlorure d'argent correspondant à 0,1 coulomb/cm$^2$), la face chlorurée de chaque électrode étant tournée du côté de la membrane en peau de rat.

Les échantillons de peau de rat étaient préparés comme indiqué dans l'exemple 1.

Pour chacun des essais réalisés, quatre cellules d'ionophorèse identiques (cellules A, B, C et D) étaient mises en route simultanément. La surface effective d'échange était de 2 cm$^2$ pour chaque peau.

Un générateur de courant pulsé permettait d'établir simultanément entre les électrodes des quatre cellules montées en parallèle un signal électrique du type potentiométrique ayant une tension crête égale à 2 volts avec un rapport cyclique de 70 % et une fréquence de 10 kHz. Une résistance de 100 ohms était branchée en série dans le circuit.

Le suivi de la tension aux bornes de cette résistance permettait de déterminer l'instant à partir duquel le chlorure d'argent était consommé en totalité, ledit instant étant indiqué par une chute brutale de ladite tension.

Le courant pulsé généré par le générateur était appliqué pendant 6 heures, l'électrode du compartiment donneur de chaque cellule contenant le valproate de sodium étant reliée au pôle négatif dudit générateur et les contre-électrodes des cellules au pôle positif.

Au bout de ladite durée, on prélevait le milieu contenu dans le compartiment receveur de chaque cellule et déterminait, par dosage, la quantité de valproate de sodium ayant traversé la peau séparant les compartiments donneur et receveur de chaque cellule.

Quatre essais 2a à 2d ont été réalisés comme suit :

. Essai 2a : Aucun courant n'a été appliqué aux électrodes, dans le but de déterminer la diffusion transcutanée passive sur 6 heures.
. Essai 2b : L'électrode d'argent chloruré présente dans le compartiment donneur de chaque cellule renfermait une quantité d'AgCl correspondant à 30 coulombs, c'est-à-dire très supérieure à la quantité susceptible d'être consommée pendant la durée de mise en oeuvre (6 heures) de l'opération d'ionophorèse (électrode non limitante).
. Essais 2c et 2d : L'électrode d'argent chloruré présente dans le compartiment donneur de chaque cellule renfermait une quantité d'AgCl correspondant respectivement à 6 coulombs (essai 2c) et 12 coulombs (essai 2d), c'est-à-dire inférieure à la quantité susceptible d'être consommée pendant la durée de mise en oeuvre (6 heures) de l'opération d'ionophorèse (électrode limitante).

Pour chacun des essais, on a déterminé pour chaque cellule :

- la quantité totale Q de valproate ayant diffusé en 6 heures dans le compartiment receveur ;
- la durée de réversibilité de l'électrode du compartiment donneur, c'est-à-dire la durée de l'opération d'ionophorèse dans le cas des essais 2a et 2b sans électrode limitante ou bien la durée au bout de laquelle le chlorure d'argent de l'électrode limitante a été consommé dans le cas des essais 2c et 2d avec électrode limitante ;
- le flux F représentant le quotient de la quantité Q par la durée d'activité et par la surface de l'électrode ; et
- le rapport Q/n de la quantité Q à la quantité de chlorure d'argent consommée, cette dernière quantité étant exprimée en coulombs.

Les résultats obtenus sont rassemblés dans le tableau II.

TABLEAU II

| Essai 2a | Diffusion transcutanée passive sur 6 heures | | | | | | |
|---|---|---|---|---|---|---|---|
| | Cell.A | Cell.B | Cell.C | Cell.D | Moyenne | Ecart type | %de variation |
| Q (µg) | 160 | 764 | 518 | 836 | 569 | 264 | 46,4 % |
| F(µg/cm$^2$.h) | 13,3 | 63,6 | 43 | 69,7 | 47,4 | 22 | 46,4 % |
| **Essai 2b** | Ionophorèse sur 6 heures -Electrodes réversibles non limitantes | | | | | | |
| | Cell.A | Cell.B | Cell.C | Cell.D | Moyenne | Ecart type | %de variation |
| Q (µg) | 1533 | 933 | 3118 | 2988 | 2170 | 964 | 44 % |
| F(µg/cm$^2$.h) | 128,5 | 77,6 | 267 | 248 | 182 | 78,8 | 44 % |
| **Essai 2c** | Ionophorèse sur 6 heures -Electrodes Ag/AgCl à 6 C d'AgCl | | | | | | |
| | Cell.A | Cell.B | Cell.C | Cell.D | Moyenne | Ecart type | %de variation |
| Q (µg) | 1127 | 1152 | 994 | 1000 | 1068 | 71,8 | 6,7 % |
| Durée d'activité (h) | 1,75 | 2,75 | 3,16 | 2,5 | 2,55 | 0,75 | 24 % |
| F (µg/cm$^2$.h) | 322 | 209 | 157 | 200 | 222 | 61 | 28 % |
| Q/n (µg/C) | 188 | 192 | 165 | 166 | 178 | 12,1 | 6,8 % |
| **Essai 2d** | Ionophorèse sur 6 heures -Electrodes Ag/AgCl à 12 C d'AgCl | | | | | | |
| | Cell.A | Cell.B | Cell.C | Cell.D | Moyenne | Ecart type | %de va-riation |
| Q (µg) | 2273 | 2158 | 2242 | 2350 | 2256 | 68,8 | 3,0 % |
| Durée d'activité (h) | 2,75 | 3,5 | 3,33 | 4,25 | 3,46 | 0,53 | 15,5 % |
| F (µg/cm$^2$.h) | 413 | 308 | 337 | 276 | 333 | 50,6 | 15 % |
| Q/n (µg/C) | 189 | 180 | 187 | 196 | 188 | 6 | 3,0 % |

La lecture des résultats obtenus dans les exemples 1 et 2 et présentés respectivement dans les tableaux I et II montre clairement que les flux ainsi que les quantités diffusées par voie passive (Tableau I, Essai la et Tableau II, Essai 2a) sont très peu reproductibles, comme le montrent les pourcentages de variation qui vont de 46,4 à 50 %. Il en va de même pour les quantités diffusées par ionophorèse utilisant des électrodes non limitantes, lorsqu'on applique aux électrodes un signal à voltage crête constant pendant une durée identique (tableau I, essai 1b et Tableau II, essai 2b). Ceci est dû essentiellement à la grande variation de structure de la peau, qui se traduit par des diffusions passives et des impédances très différentes d'un individu à l'autre.

La lecture des résultats obtenus selon l'invention (tableau I, essais 1c à 1e et tableau II, essais 2c et 2d) démontre que, malgré des courants ionophorétiques très variables d'une expérience à l'autre comme l'indiquent les durées très variables d'utilisation des électrodes réversibles ayant la même capacité faradique (c'est-à-dire renfermant la même quantité d'AgCl), les quantités totales de principe actif délivrées en 6 heures sont directement proportionnelles à la charge des électrodes, c'est-à-dire en fait à la quantité totale de courant consommée par le système, et ceci avec un pourcentage de variation 5 à 10 fois plus faible qu'avec les procédés classiques. Ceci signifie que les rendements électriques, c'est-à-dire les rapports des quantités de principe actif ayant diffusé aux quantités de courant ayant été utilisées dans le processus d'ionophorèse (Q/n), sont bien reproductibles.

Les électrodes limitantes selon l'invention se comportent comme des compteurs chimiques capables de limiter la quantité totale de courant ayant été administrée et d'entraîner, sans aucun dispositif électronique compliqué et/ou onéreux, une coupure ou tout au moins une très forte diminution du courant lorsque la quantité prédéterminée de courant, correspondant à la quantité totale donnée de principe actif à administrer, a été atteinte.

Il faut remarquer de plus que cet effet a été obtenu en appliquant entre les électrodes, et par là même à la peau, des voltages crêtes constants particulièrement aisés à obtenir qu'ils soient continus ou pulsés. Cette approche est la plus sûre car elle interdit, lorsque les tensions appliquées sont choisies convenablement, toute réaction secondaire d'oxydation ou de réduction du principe actif, du milieu ambiant ou de la peau, pouvant entraîner l'apparition de produits secondaires toxiques ou mal tolérés.

Pour obtenir le même contrôle de la quantité de courant distribuée avec des dispositifs dont la capacité des électrodes n'est pas ajustée (exemple 1, essai 1b et exemple 2, essai 2b), il faudrait soit mettre en place un dispositif de comptage (coulomètre) de la quantité de courant délivrée par le dispositif à voltage imposé soit contrôler strictement la durée d'application du traitement lorsque le dispositif fournit un courant d'intensité constante. Dans le premier cas, le dispositif électronique est onéreux et délicat à réaliser, surtout dans le cas de courants pulsés, mais peu dangereux alors que dans le second cas, il peut arriver qu'à la suite de mauvais contacts ou d'une impédance anormalement élevée de certains sujets, le voltage atteint par le dispositif s'élève exagérément et devienne dangereux soit au niveau de la tolérance cutanée soit par apparition de réactions de destruction incontrôlée du ou des principes actifs. Il est donc indispensable d'incorporer à de tels dispositifs, en plus de l'interrupteur chronométrique un dispositif limitant la tension aux électrodes. D'où un coût supplémentaire assorti d'un risque de moins bonne fiabilité.

EXEMPLE 3 :

Etude du passage transdermique du valproate de sodium avec contre-électrode limitante à base d'aluminium

On opérait dans des cellules d'ionophorèse ayant chacune une structure identique à celle des cellules utilisées dans l'exemple 1. Dans chaque cellule, le compartiment donneur (compartiment cathodique) était rempli d'une solution aqueuse de valproate de sodium à 10 % en poids, ladite solution étant additionnée de 500 ppm de $NaN_3$ à titre de biocide. Le compartiment receveur ou de contre-électrode (compartiment anodique) était rempli de sérum physiologique tamponné à pH 7. A son extrémité opposée au compartiment receveur, le compartiment donneur était équipé d'une électrode en argent chloruré ayant une surface active de 2 $cm^2$ et renfermant une quantité d'AgCl correspondant à 4 coulombs/$cm^2$. Le compartiment de contre-électrode était équipé d'une électrode de 2 $cm^2$ découpée dans une feuille de polypropylène revêtue d'un dépôt d'aluminium d'épaisseur égale à 200 nm, cette feuille de polypropylène aluminisée étant un produit commercial. La quantité d'aluminium présente sur la contre-électrode correspondait à 0,6 coulomb/$cm^2$. Le contact était pris à l'aide de pinces à mâchoires disposées sur une partie du revêtement métallique préalablement recouverte d'un vernis conducteur électronique du type époxyde à l'argent. La face chlorurée de l'électrode du compartiment donneur et le dépôt d'aluminium de l'électrode limitante du compartiment receveur étaient tournés du côté de la membrane en peau de rat.

Les échantillons de peau de rat étaient préparés comme indiqué dans l'exemple 1.

Dans cet exemple, quatre cellules d'ionophorèse identiques (cellules A, B, C et D) étaient utilisées. La surface effective d'échange était de 2 $cm^2$ pour chaque peau.

A l'aide d'un appareil commercialisé sous le nom de PHORESOR [R], on imposait aux électrodes de chacune des quatre cellules d'ionophorèse un courant continu ayant une intensité constante quelle que soit la tension que cette intensité imposée crée entre l'anode et la cathode de chaque cellule, ladite intensité imposée étant égale à 0,6mA (densité de courant égale à 0,3mA/$cm^2$).

Un coulomètre, monté dans le circuit électrique de la cellule, mesurait directement en coulombs la quantité de courant continu ayant traversé l'ensemble du circuit, tandis qu'un voltmètre, monté en dérivation, indiquait la valeur de la tension entre l'anode et la cathode de la cellule.

Le courant était appliqué de telle sorte que l'électrode du compartiment donneur de chaque cellule contenant le valproate de sodium était reliée au pôle négatif du générateur de courant associé et la contre-électrode de la cellule au pôle positif dudit générateur.

A partir de son démarrage, chaque cellule fonctionnait jusqu'à la coupure du courant déclenchée par la consommation totale de l'anode en aluminium déposé sur le film de polypropylène. Six heures après le démarrage, on prélevait le milieu contenu dans le compartiment receveur de chaque cellule et déterminait, par dosage, la quantité de valproate de sodium ayant traversé la peau séparant les compartiments donneur et receveur de chaque cellule.

Pour chacune des cellules, les grandeurs suivantes ont été déterminées :

- quantité de courant K lue sur le coulomètre équipant le circuit électrique de la cellule ;
- tension moyenne V mesurée entre les électrodes de la cellule ;
- laps de temps t entre le démarrage de l'expérience et la coupure du courant déclenchée par la consommation totale de l'aluminium de l'anode ;
- quantité totale Q de valproate ayant diffusé dans le compartiment receveur au cours de l'expérience ; et
- rapport Q/K de la quantité Q à la quantité de courant K exprimant en coulombs la quantité d'aluminium consommée.

Les résultats obtenus sont rassemblés dans le tableau III.

TABLEAU III

| | K (Coulomb) | V (volts) | t (s) | Q (µg) | Q/K (µC) |
|---|---|---|---|---|---|
| Cell. A | 1,296 | 0,93 | 2160 | 336 | 259 |
| Cell. B | 1,332 | 2,48 | 2220 | 352 | 264 |
| Cell. C | 1,116 | 3,3 | 1860 | 307 | 275 |
| Cell. D | 1,080 | 1,78 | 1800 | 268 | 248 |
| Moyenne | 1,210 | 2,12 | 2010 | 316 | 261,5 |
| Ecart type | 1,110 | 0,87 | 3,04 | 32 | 9,7 |
| % de variation | 9,1 % | 41 % | 9,1 % | 10 % | 3,7 % |

La lecture des résultats consignés dans le tableau III amène les remarques suivantes :

. toutes les électrodes à base d'aluminium ont été découpées dans un même échantillon de la feuille de polypropylène aluminisé et se révèlent comme ayant des capacités coulombiques très voisines, ce qui montre que, contre toute attente, cette qualité de feuille de polypropylène aluminisé permet la consommation de la totalité du dépôt d'aluminium sur la face en contact avec l'électrolyte ;

. la durée des expériences est assez bien reproductible, ce qui démontre que l'électrode a bien fonctionné comme un compteur électrochimique de la quantité de courant consommée ;

. on observait des écarts importants au niveau des tensions mesurées entre les électrodes. Ces écarts sont dus à des différences importantes d'impédance de la peau d'un essai à l'autre. On conçoit que ces tensions puissent dans certains cas atteindre des valeurs telles qu'elles soient mal tolérées ou qu'elles entraînent des réactions secondaires indésirables pour peu que l'on impose des courants plus élevés ou que les contacts entre la peau et le réservoir de principe actif et/ou l'électrode associée au principe actif soient défectueux ;

. malgré ces importantes variations de tension, les quantités de principe actif ayant traversé la peau au cours de chaque expérience sont bien reproductibles et parfaitement corrélées à la capacité de chaque électrode limitante ;

. les électrodes limitantes, c'est-à-dire à capacité coulombique contrôlée, à base d'aluminium ont donc fonctionné avec succès comme un compteur chronométrique dans ce cas de montage intensiostatique et ceci en toute sécurité et sans appareillage électronique ou mécanique susceptible d'accroître le coût des dispositifs et de les alourdir s'ils sont destinés à être portés à même la peau par les utilisateurs potentiels.

EXEMPLE 4 :

Etude du passage transdermique du valproate de sodium avec contre-électrode limitante à base d'aluminium et tension pulsée imposée

On opérait dans des cellules d'ionophorèse ayant chacune une structure identique à celle des cellules utilisées dans l'exemple 1. Dans chaque cellule le compartiment donneur (compartiment cathodique) était rempli d'une solution aqueuse de valproate de sodium à 10 % en poids, ladite solution étant additionnée de 500 ppm de $NaN_3$ à titre d'agent bactéricide, tandis que le compartiment receveur ou compartiment de contre-électrode était rempli de sérum physiologique tamponné à pH 7. A son extrémité opposée au compartiment receveur, le compartiment donneur était équipé d'une électrode en argent chloruré ayant une surface active de 2 $cm^2$ et renfermant une quantité de chlorure d'argent correspondant à 4 coulombs/$cm^2$. Le compartiment de contre-électrode était équipé d'une électrode de 2 $cm^2$ découpée dans une feuille de polypropylène revêtue d'un dépôt d'aluminium d'épaisseur égale à 200 nm, la quantité d'aluminium présente sur la contre-électrode correspondant à 0,6 coulomb/$cm^2$. Le contact était pris à l'aide de pinces à mâchoires disposées sur une partie du revêtement métallique recouverte d'un vernis conducteur électronique du type époxyde à l'argent. La face chlorurée de l'électrode du compartiment donneur et le dépôt d'aluminium de l'électrode limitante du compartiment receveur étaient tournés du côté de la membrane en peau de rat.

Les échantillons de peau de rat étaient préparés comme indiqué dans l'exemple 1.

Dans la réalisation de cet exemple, quatre cellules d'ionophorèse identiques (cellules A, B, C et D) étaient mises en route simultanément. La surface effective d'échange était de 2 $cm^2$ pour chaque peau.

Un générateur de courant pulsé permettait d'établir simultanément entre les électrodes des quatre cellules montées en parallèle un signal électrique du type potentiométrique ayant une tension crête égale à 3 volts avec un rapport cyclique de 50 % et une fréquence de 30 kHz. Une résistance de 100 ohms était branchée en série dans le circuit.

Le suivi de la tension aux bornes de cette résistance permettait de déterminer l'instant à partir duquel l'aluminium de l'électrode limitante était consommé en totalité, ledit instant étant indiqué par une chute brutale de ladite tension.

Le courant pulsé fourni par le générateur était appliqué pendant 6 heures, l'électrode du compartiment donneur de chaque cellule contenant le valproate de sodium étant reliée au pôle négatif dudit générateur et les contre-électrodes des cellules au pôle positif.

Au bout de ladite durée, on prélevait le milieu contenu dans le compartiment receveur de chaque cellule et déterminait, par dosage, la quantité de valproate de sodium ayant traversé la peau séparant les compartiments donneur et receveur de chaque cellule.

Pour chacune des cellules, les grandeurs suivantes ont été déterminées :

- quantité totale Q de valproate ayant diffusé en 6 heures (durée de l'expérience) dans le compartiment receveur ;
- durée t pendant laquelle le courant a une valeur significative pour l'ionophorèse, c'est-à-dire la durée au bout de laquelle l'aluminium de la contre-électrode a été consommé en totalité ;
- flux F représentant le quotient de la quantité Q par la durée t et par la surface de l'électrode ;
- rapport Q/n de la quantité Q à la quantité d'aluminium consommée, cette dernière quantité étant exprimée en coulombs et prise égale à 1,21 (valeur moyenne trouvée dans l'exemple 3).

Les résultats obtenus sont consignés dans le tableau IV.

### TABLEAU IV

| | Q ($\mu$g) | t (min) | F ($\mu$g/cm$^2$.h) | Q/n ($\mu$g/C) |
|---|---|---|---|---|
| Cellule A | 327 | 40 | 245,5 | 270,2 |
| Cellule B | 321 | 55 | 175,1 | 265,3 |
| Cellule C | 295 | 60 | 147,5 | 243,8 |
| Cellule D | 301 | 72 | 125,4 | 248,8 |
| Moyenne | 311 | 56,75 | 173,4 | 257 |
| Ecart type | 13 | 11,5 | 45 | 11 |
| % de variation | 4,3 % | 20,2 % | 26 % | 4,3 % |

La lecture des résultats présentés dans le tableau IV conduit aux conclusions suivantes :

- la durée des expériences est très variable et dépend à l'évidence de l'impédance de chaque échantillon de peau ;
- les flux transdermiques F sont très variables d'une expérience à l'autre ;
- malgré des durées t d'activité du signal très différentes, on peut constater que les quantités Q délivrées ainsi que les rapports Q/n sont très reproductibles comme le traduisent les faibles écarts types correspondant aux valeurs de Q et de Q/n ;
- les rapports Q/n sont du même ordre de grandeur que les rapports Q/K de l'exemple 3 bien que les courants appliqués soient complètement différents.

EXEMPLE 5 :

Etude du passage transdermique de valproate de sodium avec contre-électrode limitante à base d'aluminium et tension continue imposée

On opérait comme décrit dans l'exemple 4 avec toutefois les modifications suivantes :

- la solution aqueuse de valproate de sodium renfermait 15 % en poids de ce composé ;
- la contre-électrode était découpée dans une feuille de polypropylène aluminisé dont le revêtement a été enrichi en aluminium par pulvérisation d'aluminium sous vide, ladite contre-électrode ayant une épaisseur d'aluminium égale à environ 400 nm ; et
- le générateur de courant pulsé était remplacé par un générateur de courant continu délivrant une tension continue égale à 1,5 volt, un coulomètre et un milliampèremètre étant montés en série avec chaque cellule d'ionophorèse.

Pour chacune des quatre cellules A, B, C et D, les grandeurs suivantes ont été déterminées :

- quantité totale Q de valproate ayant diffusé pendant la durée de l'expérience (6 heures) dans le compartiment receveur ;
- durée t pendant laquelle le courant a une valeur significative pour l'ionophorèse, c'est-à-dire la durée au bout de laquelle l'aluminium de la contre-électrode a été consommé en totalité ;
- quantité de courant K lue sur le coulomètre au bout de la durée t ;
- intensité I moyenne du courant lue sur le milliampèremètre ;
- flux F représentant le quotient de la quantité Q par la durée t et par la surface de l'électrode ; et
- rapport Q/K de la quantité Q à la quantité K exprimant en coulombs la quantité d'aluminium consommée.

Les résultats obtenus sont rassemblés dans le tableau V.

TABLEAU V

| | K (Coulombs) | Q ($\mu$g) | t (h) | F ($\mu$g/cm$^2$.h) | Q/K ($\mu$g/C) | I (mA) |
|---|---|---|---|---|---|---|
| Cell.A | 2,92 | 814 | 3,08 | 132 | 279 | 0,133 |
| Cell.B | 2,64 | 745 | 1,75 | 212,8 | 282 | 0,208 |
| Cell.C | 2,42 | 718 | 2,08 | 186,7 | 297 | 0,160 |
| Cell. D | 2,74 | 843 | 1,08 | 389,1 | 307 | 0,351 |
| Moyenne | 2,68 | 795 | 2 | 230,1 | 292 | 0,213 |
| Ecart type | 0,18 | 36,9 | 0,72 | 96,3 | 11,7 | 0,084 |
| % de variation | 6,7 | 4,6 | 36 | 41,8 | 4,0 | 39,5 |

La lecture des résultats figurant au tableau V conduit aux conclusions suivantes :

. à tension constante, qui est le seul moyen d'éviter des réactions secondaires d'oxydoréduction néfastes, les intensités et les durées de passage du courant sont très variables d'une expérience à l'autre ;
. les quantités totales de principe actif ayant diffusé à travers la peau sont, par contre, très reproductibles ;
. les quantités ayant diffusé par coulomb sont dans cet exemple légèrement supérieures à celles déterminées pour les exemples 3 et 4, car dans le présent exemple la concentration en principe actif (valproate de sodium) dans le compartiment donneur était plus élevée.

En conclusion des exemples présentés, que le signal électrique soit pulsé ou continu, qu'il soit à tension crête imposée ou à intensité imposée, l'utilisation d'électrodes limitantes dans le système, comme le propose l'invention, permet de mieux contrôler les quantités de principe actif délivrées à travers la peau et ceci de manière fiable interdisant tout excès d'administration de principe actif et sans dispositif électronique autre que celui qui produit le signal électrique ionophorétique.

**Revendications**

1. Dispositif d'ionophorèse administrant, par voie transcutanée, une quantité totale donnée d'un principe actif à un sujet, ledit dispositif étant du type comportant un premier ensemble électrode constitué d'une première électrode, dite électrode active, en contact avec un élément réservoir, qui, d'une part, contient un électrolyte renfermant le principe actif sous une forme au moins partiellement ionisée ou sous une forme neutre et en quantité prédéterminée supérieure à la quantité totale donnée à administrer au sujet et, qui, d'autre part, est adapté pour assurer, lorsqu'il est placé au contact d'une zone de la peau du sujet, un continuum conducteur ionique entre ladite première électrode et ladite zone, la dite première électrode ayant même polarité que les ions du principe actif ou une polarité positive si le principe actif est neutre, un deuxième ensemble électrode constitué soit (i) d'une deuxième électrode, dite contre-électrode, de polarité opposée à celle associée au principe actif, ou bien de préférence (ii) d'une telle deuxième électrode en contact avec un élément récepteur renfermant un électrolyte indifférent, ledit élément récepteur étant agencé pour assurer lorsqu'il est placé au contact d'une portion de la peau du sujet, un continuum conducteur ionique entre la deuxième électrode et ladite portion, et un générateur de signaux électriques relié aux deux électrodes, la première électrode en contact avec l'élément réservoir et/ou la deuxième électrode en contact avec l'élément récepteur consistant en une électrode consommable formée d'une matière électrochimiquement consommable associée soit à un support isolant, soit à un support conducteur électronique, ledit support conducteur électronique étant réalisé en un matériau qui résiste à la corrosion par l'électrolyte associé à l'électrode en l'absence de courant et qui présente, lorsque l'électrode consommable est une électrode négative ou cathode, une surtension d'hydrogène en présence dudit électrolyte au moins égale à celle de l'aluminium ou bien qui n'est pas consommable par oxydation électrochimique lorsque l'électrode consommable est une électrode positive ou anode, l'électrode consommable ou l'une des électrodes consommables renfermant une quantité limitée de matière consommable électrochimiquement associée au support, et se caracterisant en ce que ladite quantité limitée étant choisie pour que la quantité d'électricité nécessaire à sa consommation électrochimique soit inférieure à la quantité d'électricité nécessaire pour administrer la quantité prédéterminée de principe actif renfermée dans l'électrolyte, de telle sorte que la circulation du courant entre les électrodes soit pratiquement interrompue lorsque la matière consommable de l'électrode portant la quantité limitée de matière consommable et dite électrode consommable limitante, a été consommée et de telle sorte que la quantité d'électricité nécessaire à la consommation de la quantité limitée de matière consommable puisse correspondre à la quantité d'électricité nécessaire pour administrer la quantité totale donnée de principe actif au sujet.

2. Dispositif selon la revendication 1, caractérisé en ce que la quantité de principe actif présente dans l'élément réservoir au début de l'opération est en excès d'environ 2% à 1000% et plus spécialement d'environ 2% à 500% par rapport à la quantité totale donnée de principe actif à administrer.

3. Dispositif selon la revendication 1 ou 2, caractérisé en ce que l'électrode active est l'électrode consommable limitante, tandis que la contre-électrode est une électrode non consommable électrochimiquement.

4. Dispositif selon la revendication 1 ou 2, caractérisé en ce que l'électrode active est une électrode non consommable électrochimiquement, tandis que la contre-électrode est l'électrode consommable limitante.

5. Dispositif selon la revendication 1 ou 2, caractérisé en ce que l'électrode active et la contre-électrode sont toutes les deux des électrodes consommables électrochimiquement, l'une des électrodes étant l'électrode consommable limitante, tandis que l'autre électrode est une électrode consommable non limitante, c'est-à-dire renfermant une matière consommable électrochimiquement en quantité en excès par rapport à la quantité qui serait consommée par le passage de la quantité d'électricité consommant la quantité de matière consommable électrochimiquement de l'électrode consommable limitante.

6. Dispositif selon l'une des revendications 3 à 5, caractérisé en ce que l'électrode consommable limitante ou l'électrode consommable non limitante est utilisée comme anode, la matière consommable électrochimiquement de ladite électrode étant choisie parmi les matières consommables par oxydation électrochimique et en particulier parmi les métaux tels que Al, Cu, Mg, Zn et Ag.

7. Dispositif selon la revendication 6, caractérisé en ce que l'électrode consommable de type anode est une électrode non limitante à base d'un métal choisi parmi Al, Ag, Cu, Mg et Zn, ou une électrode limitante à base d'un tel métal déposé sur un support isolant tel que polypropylène ou polyéthylène, ou sur un support conducteur électronique choisi parmi titane, acier inoxydable, platine, carbone, graphite et polymère conducteur.

8. Dispositif selon l'une des revendications 3 à 5, caractérisé en ce que l'électrode consommable limitante ou l'électrode consommable non limitante est utilisée comme cathode, la matière électrochimiquement consommable de ladite électrode étant choisie parmi les composés métalliques ionisables dont les ions métalliques sont susceptibles d'être réduits électrochimiquement en le métal correspondant et en particulier parmi les composés AgCl et CuCl.

9. Dispositif selon la revendication 8, caractérisé en ce que l'électrode consommable de type cathode est une électrode à base d'AgCl ou de CuCl, ce composé étant déposé sur un support de cuivre, d'argent, d'acier inoxydable, de titane, de carbone, de polypropylène, de polyéthylène ou d'un polymère conducteur.

10. Dispositif selon l'une des revendications 1 à 9, caractérisé en ce que le générateur de signaux électriques applique entre l'électrode active et la contre-électrode un signal intensiométrique, c'est-à-dire un signal d'intensité moyenne imposée, ou un signal potentiométrique, c'est-à-dire un signal de tension moyenne imposée, ledit signal étant continu ou pulsé et permanent ou intermittent, avec ou sans inversion temporaire de polarité, et possédant une fréquence allant de 0 à 500 kHz et plus particulièrement de 0 à 100 kHz.

**11.** Dispositif selon la revendication 10, caractérisé en ce que le signal électrique est un signal pulsé présentant un rapport cyclique, c'est-à-dire un rapport entre la durée de l'impulsion élémentaire, dont la répétition forme le signal pulsé, et l'intervalle de temps séparant deux apparitions successives de cette impulsion, allant de 0,05 à 0,95 et plus particulièrement de 0,1 à 0,8.

**12.** Dispositif selon la revendication 10 ou 11, caractérisé en ce que le signal électrique appliqué entre l'électrode active et la contre-électrode est du type potentiométrique et présente une tension moyenne comprise entre 0,1 et 50 volts et plus spécialement entre 0,5 et 20 volts de telle sorte que la densité du courant moyen généré entre lesdites électrodes ait une valeur inférieure à 5 mA/cm$^2$, plus particulièrement inférieure ou égale à 1 mA/cm$^2$ et par exemple comprise entre 0,03 et 0,5 mA/cm$^2$.

**Claims**

**1.** Iontophoresis device administering transcutaneously a given total amount of an active ingredient to a subject, the device being of the type comprising a first electrode assembly formed by a first electrode, known as the active electrode, in contact with a reservoir element, which, on the one hand, contains an electrolyte containing the active ingredient in an at least partially ionised form or in a neutral form and in a predetermined amount greater than the given total amount to be administered to the subject and which, on the other hand, is adapted to ensure, when it is placed in contact with a region of the subject's skin, an ion conductive continuum between the first electrode and said region, the first electrode having the same polarity as the ions of the active ingredient or a positive polarity if the active ingredient is neutral, a second electrode assembly formed either (i) by a second electrode, known as the counter-electrode, having an opposite polarity to that associated with the active ingredient, or preferably (ii) by such a second electrode in contact with a receiving element containing an inert electrolyte, the receiving element being arranged to ensure, when it is placed in contact with a portion of the subject's skin, an ion conductive continuum between the second electrode and said portion, and a generator of electrical signals which is connected to the two electrodes, the first electrode in contact with the reservoir element and/or the second electrode in contact with the receiving element consisting in a consumable electrode formed from an electrochemically consumable material associated either with an insulating support or with an electron conductive support, the electron conductive support being produced from a material which is resistant to corrosion by the electrolyte associated with the electrode in the absence of current and which has, when the consumable electrode is a negative electrode or cathode, a hydrogen overpotential in the presence of the electrolyte at least equal to that of aluminium or which is not consumable by electrochemical oxidation when the consumable electrode is a positive electrode or anode, the consumable electrode or one of the consumable electrodes containing a limited amount of electrochemically consumable material associated with the support, and being characterised in that said limited amount is selected so that the amount of electricity necessary for its electrochemical consumption is smaller than the amount of electricity necessary to administer the predetermined amount of active ingredient contained in the electrolyte, so that the circulation of the current between the electrodes is in practice interrupted when the consumable material of the electrode carrying the limited amount of consumable material and known as the limiting consumable electrode has been consumed and so that the amount of electricity necessary for the consumption of the limited amount of consumable material can correspond to the amount of electricity necessary to administer the given total amount of active ingredient to the subject.

**2.** Device according to Claim 1, characterised in that the amount of active ingredient present in the reservoir element at the beginning of the operation is in an excess of approximately from 2% to 1000% and more especially approximately from 2% to 500% relative to the given total amount of active ingredient to be administered.

**3.** Device according to Claim 1 or 2, characterised in that the active electrode is the limiting consumable electrode, while the counter-electrode is an electrode that is not electrochemically consumable.

**4.** Device according to Claim 1 or 2, characterised in that the active electrode is an electrode that is not electrochemically consumable, while the counter-electrode is the limiting consumable electrode.

**5.** Device according to Claim 1 or 2, characterised in that the active electrode and the counter-electrode are both electrochemically consumable electrodes, one of the electrodes being the limiting consumable electrode while the other electrode is a non-limiting consumable electrode, that is to say, containing an electrochemically consumable material in an amount in excess relative to the amount which would be consumed as a result of the passage of the amount of electricity consuming the amount of electro-chemically consumable material of the limiting consumable electrode.

6. Device according to any one of Claims 3 to 5, characterised in that the limiting consumable electrode or the non-limiting consumable electrode is used as an anode, the electrochemically consumable material of the electrode being selected from materials consumable by electrochemical oxidation and especially from metals such as Al, Cu, Mg, Zn and Ag.

7. Device according to Claim 6, characterised in that the consumable electrode of the anode type is a non-limiting electrode based on a metal selected from Al, Ag, Cu, Mg and Zn, or a limiting electrode based on such a metal deposited on an insulating support, such as polypropylene or polyethylene, or on an electron conductive support selected from titanium, stainless steel, platinum, carbon, graphite and conductive polymer.

8. Device according to any one of Claims 3 to 5, characterised in that the limiting consumable electrode or the non-limiting consumable electrode is used as a cathode, the electrochemically consumable material of the electrode being selected from ionisable metal compounds of which the metal ions are capable of being reduced electrochemically to the corresponding metal and especially from the compounds AgCl and CuCl.

9. Device according to Claim 8, characterised in that the consumable electrode of the cathode type is an electrode based on AgCl or CuCl, this compound being deposited on a support of copper, silver, stainless steel, titanium, carbon, polypropylene, polyethylene or a conductive polymer.

10. Device according to any one of Claims 1 to 9, characterised in that the generator of electrical signals applies between the active electrode and the counter-electrode an intensiometric signal, that is to say, a signal of set average intensity, or a potentiometric signal, that is to say, a signal of set average voltage, the signal being continuous or pulsed and permanent or intermittent, with or without temporary inversion of polarity, and having a frequency of from 0 to 500 kHz and more especially from 0 to 100 kHz.

11. Device according to Claim 10, characterised in that the electrical signal is a pulsed signal having a cyclic ratio, that is to say, a ratio of the duration of the elementary pulse, the repetition of which forms the pulsed signal, to the time interval separating two consecutive appearances of that pulse, ranging from 0.05 to 0.95 and more especially from 0.1 to 0.8.

12. Device according to Claim 10 or 11, characterised in that the electrical signal applied between the active electrode and the counter-electrode is of the potentiometric type and has an average voltage of between 0.1 and 50 volts and more especially between 0.5 and 20 volts, so that the density of the average current generated between the electrodes has a value of less than 5 mA/cm$^2$, more especially less than or equal to 1 mA/cm$^2$ and, for example, between 0.03 and 0.5 mA/cm$^2$.

**Patentansprüche**

1. Iontophoresevorrichtung zum transdermalen Verabreichen einer vorgegebenen Gesamtmenge eines Wirkstoffes an ein Lebewesen, wobei die Vorrichtung des Typs ist, der die folgenden Elemente aufweist: eine eine erste Elektrode aufweisende erste Elektrodeneinheit, die sogenannte aktive Elektrode, die mit einem Reservoir in Kontakt steht, in dem sich einerseits ein den Wirkstoff in wenigstens teilweise ionisierter Form oder in neutraler Form und in einer vorbestimmten, die vorgebene, dem Lebewesen zu verabreichende Gesamtmenge übersteigenden Menge enthaltender Elektrolyt befindet, und das andererseits so beschaffen ist, daß beim Kontakt mit einer Hautzone des Lebewesens ein ionisches Leiterkontinuum zwischen der ersten Elektrode und der Zone gebildet wird, wobei die erste Elektrode die gleiche Polarität wie die Ionen des Wirkstoffs aufweist, oder eine positive Polarität, wenn der Wirkstoff neutral ist, eine zweite Elektrodeneinheit, die entweder (i) eine zweite Elektrode, die sogenannte Gegenelektrode, mit einer der mit dem Wirkstoff verbundenen Elektrode entgegengesetzten Polarität, oder vorzugsweise (ii) eine solche zweite Elektrode aufweist, die mit einem einen neutralen Elektolyten enthaltenden Rezeptor in Kontakt steht, wobei der Rezeptor so beschaffen ist, daß er beim Kontakt mit einem Teil der Haut des Lebewesens zwischen der zweiten Elektrode und dem Hautteil ein ionisches Leiterkontinuum herstellt, und eine mit den beiden Elektroden verbundene elektrische Signalerzeugungseinrichtung, wobei die erste mit dem Reservoir und/oder die zweite, mit dem Rezeptor in Kontakt stehende Elektrode eine selbstverzehrende Elektrode ist, die aus einem sich elektrochemisch zersetzenden Material ist und entweder mit einem isolierenden Träger oder mit einem elektronisch leitfähigen Träger verbunden ist, wobei der elektronisch leitfähige Träger aus einem Material gebildet ist, das der Korrosion durch den mit der Elektrode verbundenen Elektrolyten in der Abwesenheit von elektrischem Strom widersteht und die, wenn die verzehrbare Elektrode eine negative Elektrode oder Kathode ist, in der Gegenwart des Elektrolyten ein auf Wasserstoff bezogenes Potential hat, das mindestens gleich demjenigen von Aluminium ist oder die nicht durch elektrochemische Oxidation zersetzbar ist, wenn die selbsverzehrende Elektrode eine positive Elektrode oder Anode ist, wobei die selbstverzehrende Elektrode oder eine der selbstverzehrenden Elektroden eine begrenzte Menge sich elektrochemisch zersetzenden Materials auf dem Träger aufweist; und dadurch gekennzeichnet ist, daß die begrenzte Menge so gewählt wurde, daß die zu deren elektrochemischer Zersetzung erforderliche Elektrizitätsmenge unter der Elektrizitätsmenge liegt, die zur Verabreichung der im Elektrolyt enthaltenen vorbestimmten Menge des Wirkstoffs ist, so daß der Stromkreis zwischen den Elektroden praktisch unterbrochen wird, wenn das sich zersetzende Material der die begrenzte Menge der sich zersetzenden Substanz enthaltenden Elektrode, der sogenannten selbstverzehrenden begrenzenden Elektrode, sich zersetzt hat, und damit die zur Zersetzung der begrenzten Menge des zersetzbaren Materials erforderliche Elektrizitätsmenge der zum Verabreichen der vorgegebenen Gesamtmenge des Wirkstoffs an das Lebewesen erforderlichen Elektrizitätsmenge entsprechen kann.

2. Vorrichtung nach Anspruch 1, dadurch gekennzeichnet, daß am Anfang des Betriebs die im Reservoir vorhandene Wirkstoffmenge über ungefähr 2% bis 1000% und insbesondere ungefähr 2% bis 500% im Vergleich zur zu verabreichenden Gesamtwirkstoffmenge ist.

3. Vorrichtung nach Anspruch 1 oder 2, dadurch gekennzeichnet, daß die aktive Elektrode die selbstverzehrende begrenzende Elektrode ist, während die Gegenelektrode eine elektrochemisch nicht zersetzbare Elektrode ist.

4. Vorrichtung nach Anspruch 1 oder 2, dadurch gekennzeichnet, daß die aktive Elektrode eine elektrochemisch nicht zersetzbare Elektrode ist, während die Gegenelektrode die selbstverzehrende begrenzende Elektrode ist.

5. Vorrichtung nach Anspruch 1 oder 2, dadurch gekennzeichnet, daß die aktive Elektrode und die Gegenelektrode beide elektrochemisch selbstverzehrende Elektroden sind, wobei die eine der Elektroden die selbstverzehrende begrenzende Elektrode ist, während die andere Elektrode eine selbstverzehrende nicht begrenzende Elektrode ist, das heißt ein elektrochmisch zersetzbares Material in einer Menge enthält, die größer ist als die Menge, die durch das Hindurchgehen der das elektrochemisch zersetzbare Material der selbstverzehrenden begrenzenden Elektrode zersetzenden Elektrizitätsmenge zersetzt wird.

6. Vorrichtung nach einem der Ansprüche 3 bis 5, dadurch gekennzeichnet, daß die selbstverzehrende begrenzende Elektrode oder die selbstverzehrende nicht begrenzende Elektrode als eine Anode verwendet wird, wobei das elektrochemisch zersetzbare Material dieser Elektrode aus den durch elektrochemische Oxydation zersetzbaren Substanzen und insbesondere aus den Metallen ausgewählt ist, wie zum Beispiel Al, Cu, Mg, Zn und Ag.

7. Vorrichtung nach Anspruch 6, dadurch gekennzeichnet, daß die selbstverzehrende Elektrode des Anodentyps eine nicht begrenzende Elektrode und aus einem Metall ist, das aus der Gruppe Al, Ag, Cu, Mg und Zn ausgewählt ist, oder eine begrenzende Elektrode aus einem solchen auf einem isolierenden Träger, zum Beispiel aus Polypropylen oder Polyethylen, oder auf einem elektronisch leitfähigen Träger aufgebrachten Metall, wobei der Träger aus einem Material der folgenden Gruppe sein kann: Titan, nichtrostender Stahl, Platin, Kohlenstoff, Graphit und leitfähiges Polymer.

8. Vorrichtung nach einem der Ansprüche 3 bis 5, dadurch gekennzeichnet, daß die selbstverzehrende begrenzende Elektrode oder die selbstverzehrende nicht begrenzende Elektrode als eine Kathode verwendet wird, wobei das elektrochemisch zersetzbare Material dieser Elektrode aus den ionisierbaren Metallverbindungen ausgewählt ist, deren Metallionen elektrochemisch in das entsprechende Metall reduziert werden können, und insbesondere aus den Verbindungen AgCl und CuCl.

9. Vorrichtung nach **Anspruch 8**, dadurch gekennzeichnet, daß die selbstverzehrende Elektrode des Kathodentyps eine Elektrode aus AgCl oder CuCl ist, wobei diese Verbindung auf einem Träger aus Kupfer, Silber, nichtrostendem Stahl, Titan, Kohlenstoff, Polypropylen, Polyethylen oder aus leitfähigem Polymer abgelagert ist.

10. Vorrichtung nach einem der Ansprüche 1 bis 9, dadurch gekennzeichnet, daß die elektrische Signalerzeugungseinrichtung zwischen der aktiven Elektrode und der Gegenelektrode ein intensiometrisches Signal, das heißt ein Signal mit einer erzwungenen durchschnittlichen Stromstärke, oder ein potentiometrisches Signal, das heißt ein Signal mit einer erzwungenen durchschnittlichen Spannung ist, wobei das Signal kontinuierlich oder pulsierend und permanent oder unterbrochen sein kann, mit oder ohne zeitweise Umpolung, und mit einer Frequenz zwischen 0 und 500 kHz und insbesondere zwischen 0 und 100 kHz.

11. Vorrichtung nach **Anspruch 10**, dadurch gekennzeichnet, daß das elektrische Signal ein pulsierendes Signal mit einem Taktverhältnis ist, das heißt mit einem Verhältnis zwischen der Dauer des Grundimpulses, dessen Wiederholung das pulsierende Signal bildet, und dem zwei aufeinanderfolgende Erscheinungen dieses Impulses trennenden Zeitintervall, das zwischen 0,05 und 0,95 und insbesondere zwischen 0,1 und 0,8 liegt.

12. Vorrichtung nach Anspruch 10 oder 11, dadurch gekennzeichnet, daß das zwischen der aktiven und der Gegenelektrode angelegte elektrische Signal ein potentiometrisches Signal ist und eine Spannung von zwischen 0,1 und 50 Volt und insbesondere zwischen 0,5 und 20 Volt hat, so daß die zwischen den genannten Elektroden erzeugte durchschnittliche Stromdichte unter 5 mA/cm$^2$, insbesondere unter 1 mA/cm$^2$ und zum Beispiel zwischen 0,03 und 0,5 mA/cm$^2$ liegt.